# EUROPEAN PATENT APPLICATION

(11) **EP 1 772 143 A1**
(43) Date of publication of application: **11.04.2007**
(21) Application number: 05741624.0
(22) Date of filing: 20.05.2005
(51) Int. Cl.: A61K 31/045

(54) **PEROXISOME PROLIFERATOR-ACTIVATED RECEPTOR (PPAR) ACTIVATOR AND DRUGS, SUPPLEMENTS, FUNCTIONAL FOODS AND FOOD ADDITIVES USING THE SAME**

(30) Priority: 20.05.2004 JP 2004150667; 23.08.2004 JP 2004242653
(71) Applicant: Arkray, Inc., Kyoto-shi, Kyoto 601-8045 (JP)
(72) Inventor: SASAKI, Takao c/o Arkray, Inc., Kyoto-shi, Kyoto 601-8045 (JP)
(74) Representative: Matthews, Derek Peter
(86) International application number: PCT/JP2005/009258
(87) International publication number: WO 2005/112904

(57) **Abstract**

It is intended to provide a peroxisome proliferator-activated receptor (PPAR) activator, which is free from the problem of side effects, can be taken over a long term and has no characteristics taste. β-cryptoxantine is employed as a PPAR activator. β-cryptoxantine, which is contained in a large amount in the pulp of citrus fruits (in particular, mandarin-type citrus fruits) such as satsuma oranges, for example, has been consumed for many years. Thus, it is free from any problem in safety and has a low calorie content. Therefore, it can be taken over a long term. Because of being tasteless and odorless, moreover, β-cryptoxantine would not damage the unique taste when added to a food. Therefore, it can be added to foods and taken.

## Description

### Technical Field

The present invention relates to a peroxisome proliferator-activated receptor (PPAR) activator, and drugs, supplements, functional foods and food additives using the same.

### Background Art

The development of diabetes is said to be associated with two factors, namely, a decrease in insulin secretion and an insulin resistance. Recently, a greater number of Japanese people have become afflicted with the diabetes. Since the decrease in insulin secretion mostly is attributable to genetic factors, it is considered that a major cause of the increase in the number of diabetics is not the decrease in insulin secretion but the insulin resistance. Such an insulin resistance reportedly is caused by an increase in fat intake due to westernized dietary habits of Japanese people as well as lack of exercise, obesity and stress. Recent studies have revealed that the mechanism of the occurrence of insulin resistance is ascribable to hypertrophic fat cells. In other words, hypertrophic fat cells cause TNF-α and free fatty acid (FFA) to be secreted, thus not only impairing the sugar intake in muscle cells and liver cells but also inhibiting the secretion of adiponectin, which promotes a function of insulin, so that the insulin resistance occurs.

On the other hand, studies of the insulin resistance have shown that the activation of PPARs, which are intranuclear receptors, is effective in relieving the insulin resistance. PPARs are known to have three types, i.e., α, σ and γ, and several subtypes. PPARα is expressed mainly in the liver cells and also in other cells such as myocardial cells and gastrointestinal cells, and concerned with fatty acid oxidation, ketogenesis and apolipoprotein generation. Although PPARσ is not considered to have tissue specificity and is expressed throughout the body, it is expressed notably in large intestinal cancer cells. PPARγ can be classified into two subtypes, i.e., type γ1 and type γ2. The type γ1 is expressed in adipose tissues, immune system tissues, the adrenal gland and the small intestine, whereas the type γ2 is expressed specifically to fat cells and plays an important role in differentiation induction of the fat cells and fat synthesis.

As described above, PPARs greatly are involved with the relief of insulin resistance. In addition, PPARs are said to be concerned with the relief of hyperinsulinism, type 2 diabetes as well as obesity, hypertension, hyperlipemia and arteriosclerosis. From this viewpoint, studies have been conducted on substances that activate PPARs, and synthetic substance-based PPAR activators such as fibrate-based compound, thiazolidines, fatty acids, leukotriene B4, indomethacin, ibuprofen, fenoprofen, 15-deoxy-Δ-12,14-PGJ2 are known, for example. However, since such synthetic substance-based PPAR activators have a problem of side effects caused by long-term intake, they are not suitable for preventing or relieving diseases such as the insulin resistance by daily intake. Other than the above, natural substances such as curcumin contained in turmeric, monoacylglycerol, which is one kind of fats and oils, catechin contained in tea, etc. have been reported as PPAR activators derived from natural components (see Patent document 1, for example). However, fats and oils have a high calorie content, though they are derived from natural components, and therefore, a problem arises if they are taken continuously. Further, although it is ideal that the natural component-derived PPAR activators be added to foods or the like for daily intake, they are not suitable for the addition to foods or the like because they often have peculiar tastes.
Patent document 1: JP 2002-80362 A

### Disclosure of Invention

### Problem to be Solved by the Invention

The present invention was made with the foregoing in mind, and it is an object of the present invention to provide a PPAR activator that is free from a problem of side effects, can be taken for a long term and does not cause any problem even when added to foods or the like.

### Means for Solving Problem

In order to achieve the above-mentioned object, a PPAR activator according to the present invention contains β-cryptoxantine.

### Effects of the Invention

For the purpose of solving the problems described above, the inventor of the present invention conducted a series of studies on PPAR activators of natural components and found that β-cryptoxantine, which was contained in a large amount in mandarin-type citrus fruits such as satsuma oranges, had a PPAR activating function, thus arriving at the present invention. In other words, satsuma oranges containing a large amount of β-cryptoxantine have been eaten for many years and confirmed in terms of safety. Also, β-cryptoxantine has a low calorie content and, in this regard, does not cause any problem even if it is taken by a diabetic patient, an obese patient or the like for a long term. Further, since β-cryptoxantine is tasteless and odorless, it does not impair the unique taste of a food or the like when added to this food, so that it can be added to foods and taken daily over a long term. Therefore, in accordance with the present invention, β-cryptoxantine activates PPARs, thereby promoting fat burning, thus inhibiting the secretion of TNF-α and free fatty acid and promoting the secretion of adiponectin. Accordingly, it is possible to normalize the state of fat cells and relieve the insulin resistance and other symptoms such as hyperinsulinism, type 2 diabetes, hypertension, hyperlipemia, arteriosclerosis and obesity. It should be noted that this is effective for not only humans but also other animals.

### Brief Description of Drawings

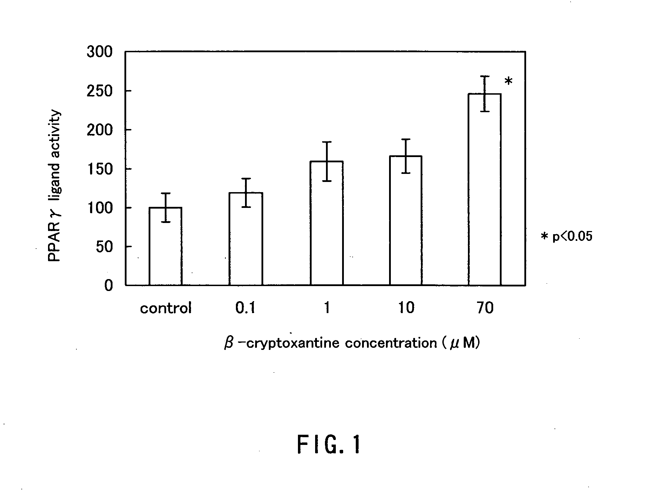

[FIG. 1] FIG. 1 is a graph showing PPARγ ligand activity of β-cryptoxantine in an example of the present invention.

### Description of the Invention

A PPAR activator according to the present invention is appropriate as long as it contains β-cryptoxantine, and also may contain components such as other PPAR activators, for example, other than β-cryptoxantine.

In the present invention, the PPAR to be activated may be either PPARα or PPARγ, for example, and preferably is both of them.

As described above, since β-cryptoxantine has a PPAR activating function, the PPAR activator according to the present invention has at least one of the functions of inhibiting the secretion of TNF-α and free fatty acid in fat cells, promoting the secretion of adiponectin in fat cells and promoting β oxidation of fat in liver cells, for example. Moreover, the PPAR activator according to the present invention has a function of inducing at least one of apoptosis, differentiation, shrinkage and the like of a fat cell, for example.

In the PPAR activator according to the present invention, the β-cryptoxantine to be used is not particularly limited, and examples thereof include those derived from citrus fruits, persimmons, papayas, loquats, red bell peppers and the like. In particular, citrus fruits are preferable. The β-cryptoxantine derived from mandarin-type citrus fruits is more preferable, and that derived from satsuma oranges is particularly preferable. This is because, since an industrial method for manufacturing β-cryptoxantine from citrus fruits has been established as described later (see, JP 3359298 B, for example), inexpensive and safe β-cryptoxantine is available. In particular, satsuma oranges contain β-cryptoxantine at a concentration as high as about 1.0 to 2.9 mg/100 g. Further, as the material, it is possible to use the entire fruit, for example, and it is particularly preferable to use the pulp. Incidentally, in the present invention, β-cryptoxantine may be a product obtained by isolation and purification from the above-noted citrus fruits or may be a commercially available product, for example.

Next, a drug according to the present invention is a drug for preventing or treating at least one disease selected from the group consisting of insulin resistance, hyperinsulinism, type 2 diabetes, hypertension, hyperlipemia, arteriosclerosis and obesity, for example, and the drug contains the PPAR activator according to the present invention. The drug of the present invention may contain not only the PPAR activator according to the present invention but also other PPAR activators and various additives, for example. In the drug according to the present invention, examples of its specific dosage form can include a tablet, a granule (including powder), a capsule, a solution (including a syrup) and the like. The drug according to the present invention can be manufactured by using an additive or a base, etc. that is suitable for the respective dosage form as necessary according to a regular method described in the Pharmacopoeia of Japan or the like. Also, a route of administration is not particularly limited but can be, for example, an oral administration or a parenteral administration. Examples of the parenteral administration can include intraoral administration, tracheobronchial administration, intrarectal administration, subcutaneous administration, intramuscular administration, intravenous administration and the like.

Now, a supplement according to the present invention is a supplement for preventing or relieving at least one disease selected from the group consisting of insulin resistance, hyperinsulinism, type 2 diabetes, hypertension, hyperlipemia, arteriosclerosis and obesity, for example, and the supplement contains the PPAR activator according to the present invention. The supplement of the present invention may contain not only the PPAR activator according to the present invention but also other PPAR activators, various additives, other supplements and the like, for example. Examples of the above-noted other supplements can include various vitamins such as vitamin C, amino acids and oligosaccharides. The supplement according to the present invention may be in any form without particular limitation, which can be, for example, tablets, fine grains (including pulvis), capsules, solution (including syrup) or the like.

Next, a functional food according to the present invention is a functional food for preventing or relieving at least one disease selected from the group consisting of insulin resistance, hyperinsulinism, type 2 diabetes, hypertension, hyperlipemia, arteriosclerosis and obesity, and the functional food contains the PPAR activator according to the present invention. The functional food of the present invention may contain not only the PPAR activator according to the present invention but also other PPAR activators, various additives and the like, for example. Incidentally, the functional food according to the present invention may be in any form without particular limitation, which can be, for example, noodles, confectionery, functional drinks or the like.

Now, a food additive according to the present invention is a food additive for preventing or relieving at least one disease selected from the group consisting of insulin resistance, hyperinsulinism, type 2 diabetes, hypertension, hyperlipemia, arteriosclerosis and obesity, and the food additive contains the PPAR activator according to the present invention. The food additive of the present invention may contain not only the PPAR activator according to the present invention but also other PPAR activators, various additives and the like, for example. The food additive according to the present invention may be in any form without particular limitation, which can be, for example, liquid, paste, powder, flakes, granule or the like. Moreover, the food additive according to the present invention includes, for example, food additives for drinks.

Next, a method for activating a PPAR according to the present invention includes, for example, bringing β-cryptoxantine into contact with a fat cell, a liver cell or the like.

The method for activating a PPAR according to the present invention induces at least one of the functions of inhibiting the secretion of TNF-α and free fatty acid in fat cells, promoting the secretion of adiponectin in fat cells and promoting β oxidation of fat in liver cells, for example. Moreover, the method for activating a PPAR according to the present invention induces at least one of apoptosis, differentiation, shrinkage and the like of a fat cell, for example.

In the method for activating a PPAR according to the present invention, the β-cryptoxantine to be used is similar to that used for the above-noted PPAR activator according to the present invention, and examples thereof include those derived from citrus fruits, persimmons, papayas, loquats, red bell peppers and the like. In particular, citrus fruits are preferable. The β-cryptoxantine derived from mandarin-type citrus fruits is more preferable, and that derived from satsuma oranges is particularly preferable. Further, as the material, it is possible to use the entire fruit, for example, and it is particularly preferable to use the pulp.

Now, a method for preventing, treating or improving a disease according to the present invention is a method for preventing, treating or improving at least one disease selected from the group consisting of insulin resistance, hyperinsulinism, type 2 diabetes, hypertension, hyperlipemia, arteriosclerosis and obesity in a mammal, and the method includes administering β-cryptoxantine. The above-noted mammal can be, for example, a human, a mouse, a rat, a rabbit, a dog, a cat, a cow, a horse, a swine, a monkey or the like.

Next, a kit according to the present invention is a kit for preventing or treating at least one disease selected from the group consisting of insulin resistance, hyperinsulinism, type 2 diabetes, hypertension, hyperlipemia, arteriosclerosis and obesity, and the kit includes
a) β-cryptoxantine,
b) a second drug composition containing a second compound useful for preventing or treating at least one disease selected from the group consisting of insulin resistance, hyperinsulinism, type 2 diabetes, hypertension, hyperlipemia, arteriosclerosis and obesity, and
c) a container for containing the β-cryptoxantine and the second drug composition.

Now, a use according to the present invention is a use of β-cryptoxantine for manufacturing a PPAR activator.

Further, a use according to the present invention is a use including administering β-cryptoxantine for preventing, treating or improving at least one disease selected from the group consisting of insulin resistance, hyperinsulinism, type 2 diabetes, hypertension, hyperlipemia, arteriosclerosis and obesity in a mammal. The mammal is as listed above.

In the use according to the present invention, the β-cryptoxantine to be used is similar to that used for the above-noted PPAR activator according to the present invention, and examples thereof include those derived from citrus fruits, persimmons, papayas, loquats, red bell peppers and the like. In particular, citrus fruits are preferable. The β-cryptoxantine derived from mandarin-type citrus fruits is more preferable, and that derived from satsuma oranges is particularly preferable. Further, as the material, it is possible to use the entire fruit, for example, and it is particularly preferable to use the pulp.

In the use according to the present invention, the β-cryptoxantine induces at least one of the functions of inhibiting the secretion of TNF-α, and free fatty acid in fat cells, promoting the secretion of adiponectin in fat cells and promoting β oxidation of fat in liver cells, for example. Moreover, in the use according to the present invention, the β-cryptoxantine induces at least one of apoptosis, differentiation, shrinkage and the like of a fat cell, for example.

Now, it is preferable that the β-cryptoxantine in the present invention is manufactured from a material such as citrus fruits as described earlier. The following is a description of an example of this manufacturing method (described in JP 3359298 B).

The β-cryptoxantine can be manufactured from citrus fruits by the method including the processes (1) to (4) below:
(1) extracting a juice from a citrus fruit and filtering or sieving the juice;
(2) centrifuging the juice at low speed to obtain supernatant and centrifuging the supernatant at high speed to obtain a precipitate;
(3) adding an enzyme for solubilizing the precipitate, followed by freezing; and
(4) after thawing and solid-liquid separating the precipitate, removing water from the precipitate to obtain solids.

Examples of the citrus fruit used in the above-described manufacturing method include a satsuma orange, an Iyo orange, a Watson pomelo, a hassaku orange, a ponkan orange, a navel orange, a lemon, a Valencia orange and a grapefruit. Among them, mandarin-type citrus fruits are preferable because of their large content of β-cryptoxantine, and a satsuma orange is more preferable. Further, although the entire citrus fruit can be used as the material, it is particularly preferable to use a pulp.

The above-noted citrus fruit usually goes through screening, washing and then extraction. An extractor is, for example, an in-line extractor, a chopper pulper extractor or a Brown extractor. Since small pieces of inner skins and bulky pulps usually are mixed in the resultant juice, the juice is filtered or sieved in order to remove them. For this filtering or sieving, a paddle-shaped finisher or a screw-shaped finisher, for example, can be used. The size of its screen mesh is 0.3 to 0.5 mm, for example.

Next, the juice is processed by centrifugation. This centrifugation processing consists of low-speed centrifugation and high-speed centrifugation under the following conditions. The low-speed centrifugation refers to centrifugation at a level capable of separating large grains of pulps. The high-speed centrifugation refers to centrifugation at a level capable of centrifuging small grains of pulps. The centrifugal intensity of the low-speed centrifugation is not greater than 3000 × g·min., for example, and that of the high-speed centrifugation is equal to or greater than 1500 × g·min., for example, so that the centrifugal intensity of the low-speed centrifugation operation is set to be lower than that of the high-speed centrifugation. Now, the juice is centrifuged at low speed, and the resultant supernatant is centrifuged at high speed further, thus collecting a precipitate.

Subsequently, a solubilizing enzyme is added to the precipitate obtained by the high-speed centrifugation. As the above-noted solubilizing enzyme, it is possible to use pectinase, cellulase, hemicellulase, protease, lipase, maceration enzymes, protopectinase and the like, for example. These enzymes may be used alone or in combination of two or more. The ratio of the above-noted solubilizing enzyme to be added ranges from 0.5 to 10 g with respect to 1 kg of the precipitate.

Thereafter, the precipitate to which the solubilizing enzyme has been added is filled in a container and frozen without warming. Then, the frozen precipitate is thawed out. The thawing may be carried out by allowing the precipitate to stand at room temperature. The thawed precipitate is solid-liquid separated, and water is removed by centrifugation so as to obtain solids (precipitate portion). These solids contain a high concentration of β-cryptoxantine. Incidentally, by repeating the operations of adding purified water to the solids and conducting centrifugation, it is possible to raise the concentration of β-cryptoxantine in the solids further.

### Example 1

The present example confirmed the activation of PPARγ by β-cryptoxantine.

First, CV-1 cells (cultured cells derived from kidneys of male African green monkeys) were implanted on 24-well culture plates so as to be 0.2 µg/well and cultured at 37°C in 5% CO₂ for 24 hours. As a medium, DMEM (Dulbecco's Modified Eagle Medium; manufactured by GIBCO) containing 10% FBS (fetal bovine serum) and a 10 mg/mL penicillin streptomycin solution was used. Next, using Lipofectamine system (manufactured by Invitrogen Corporation), pM-hPPARγ and p4×UASg-tk-luc were transfected into the cultured CV-1 cells. The above-noted pM-hPPARγ was a vector for expressing fused protein containing residues 1- 147 of GAL4 binding domain and residues 204 - 505 of human PPARγ ligand-binding domain, whereas the above-noted p4xUASg-tk-luc was a reporter plasmid containing four copies of an upstream activating sequence (UAS) for GAL4 binding domain and a thymidine kinase gene promoter in front of a luciferase gene. After the transfection, the cells were cultured for about 24 hours, and then, the media for the cells were changed to media containing β-cryptoxantine at respective concentrations (0.1, 1.0, 10 and 70 µM) or media for non-treatment control, followed by an additional 24 hour incubation. The above-noted media containing β-cryptoxantine were prepared by adding β-cryptoxantine dissolved in dimethyl sulfoxide (DMSO) to the media, whereas the media for non-treatment control were prepared by adding only DMSO to the media. After the incubation, the cells were lysed for luciferase activation assay using a Dual-Luciferase Reporter Gene Assay system (manufactured by Promega Corporation) (measurement group).

Similarly to the measurement group, as a control group, the luciferase activation assay was performed using pM (a vector containing residues 1- 147 of GAL4 binding domain and not containing residues 204 - 505 of PPARγ ligand-binding domain in pM-hPPARγ) instead of pM-hPPARγ. For each sample, the ratio between average light-emission intensities of the measurement group and the control group (n = 4) (measurement group / control group) was calculated, and a luciferase activity relative to the non-treatment control was determined as the PPARγ ligand-binding activity of the sample. Table 1 below and the graph of FIG. 1 show the results.

**[Table 1]**

| | Addition concentration | PPARγ ligand activity |
|---|---|---|
| Non-treatment control (DMSO) | (0.1%) | 100 |
| β-cryptoxantine | 0.1 µM | 119 ± 18.4 |
| | 1.0 µM | 159 ± 25.0 |
| | 10 µM | 166 ± 21.7 |
| | 70 µM | 246 ± 22.5 |
| | | (average ± standard error) |

As becomes clear from Table 1 and FIG. 1 mentioned above, the β-cryptoxantine improved the activity of PPARγ such that the PPARγ activity increased in keeping with the concentration of β-cryptoxantine.

### Industrial Applicability

As described above, the PPAR activator according to the present invention has an excellent PPAR activity, is free from a problem of side effects, can be taken over a long term and can be used preferably for foods or the like. Thus, the PPAR activator according to the present invention can be used as a drug, a supplement, a functional food and a food additive for preventing or improving diseases such as insulin resistance, hyperinsulinism, type 2 diabetes, hypertension, hyperlipemia, arteriosclerosis and obesity, etc, for example. It should be noted that this is effective for not only humans but also other animals.

## Claims

1. An activator of a peroxisome proliferator-activated receptor (PPAR), comprising β-cryptoxantine.

2. The activator for a PPAR according to claim 1, which induces at least one selected from the group consisting of apoptosis, differentiation and shrinkage of a fat cell.

3. The activator for a PPAR according to claim 1, wherein the β-cryptoxantine is derived from a mandarin-type citrus fruit.

4. The activator for a PPAR according to claim 3, wherein the mandarin-type citrus fruit is a satsuma orange.

5. A drug for preventing or treating at least one disease selected from the group consisting of insulin resistance, hyperinsulinism, type 2 diabetes, hypertension, hyperlipemia, arteriosclerosis and obesity, the drug comprising the activator of a PPAR according to claim 1.

6. A supplement for preventing or relieving at least one disease selected from the group consisting of insulin resistance, hyperinsulinism, type 2 diabetes, hypertension, hyperlipemia, arteriosclerosis and obesity, the supplement comprising the activator of a PPAR according to claim 1.

7. A functional food for preventing or relieving at least one disease selected from the group consisting of insulin resistance, hyperinsulinism, type 2 diabetes, hypertension, hyperlipemia, arteriosclerosis and obesity, the functional food comprising the activator of a PPAR according to claim 1.

8. A food additive for preventing or relieving at least one disease selected from the group consisting of insulin resistance, hyperinsulinism, type 2 diabetes, hypertension, hyperlipemia, arteriosclerosis and obesity, the food additive comprising the activator of a PPAR according to claim 1.

9. A method for activating a PPAR, comprising bringing β-cryptoxantine into contact with at least one of a fat cell and a liver cell.

10. The method according to claim 9, which induces at least one selected from the group consisting of apoptosis, differentiation and shrinkage of a fat cell.

11. The method according to claim 9, wherein the β-cryptoxantine is derived from a mandarin-type citrus fruit.

12. The method according to claim 11, wherein the mandarin-type citrus fruit is a satsuma orange.

13. A method for preventing, treating or improving at least one disease selected from the group consisting of insulin resistance, hyperinsulinism, type 2 diabetes, hypertension, hyperlipemia, arteriosclerosis and obesity in a mammal, the method comprising administering β-cryptoxantine.

14. A use of β-cryptoxantine for manufacturing a PPAR activator.

15. The use according to claim 14, wherein the β-cryptoxantine is derived from a mandarin-type citrus fruit.

16. The use according to claim 15, wherein the mandarin-type citrus fruit is a satsuma orange.

17. A use comprising administering β-cryptoxantine for preventing, treating or improving at least one disease selected from the group consisting of insulin resistance, hyperinsulinism, type 2 diabetes, hypertension, hyperlipemia, arteriosclerosis and obesity in a mammal.

18. The use according to claim 17, which induces at least one selected from the group consisting of apoptosis, differentiation and shrinkage of a fat cell.
